# EUROPEAN PATENT SPECIFICATION

(11) **EP 0 493 758 B1**
(45) Date of publication and mention of the grant of the patent: **13.03.1996**
(21) Application number: 91121925.1
(22) Date of filing: 20.12.1991
(51) Int. Cl.: C12Q 1/00, C12M 1/00, C12Q 1/37, C12Q 1/34

(54) **An instrument and a method for measuring the activity of an enzyme or the activity of an enzymatic reaction-inhibiting substance**
Ein Gerät und ein Verfahren zur Messung der Tätigkeit eines Enzyms oder der Tätigkeit einer Substanz, die eine enzymatische Reaktion hemmt
Un instrument et procédé pour la mesure de l'activité d'une enzyme ou l'activité d'une substance qui inhibe une réaction enzymatique

(30) Priority: 26.12.1990 JP 414439/90
(43) Date of publication of application: 08.07.1992
(73) Proprietor: HOECHST JAPAN LIMITED, Tokyo 100-91 (JP)
(72) Inventor: Komiyama, Osamu, Nakano-ku, Tokyo (JP); Kitagawa, Hiroshi, Hiki-Gun, Saitama-Ken (JP)
(74) Representative: Fischer, Hans-Jürgen, Dr.

(56) References cited:
- EP-A- 0 319 334
- EP-A- 0 329 190
- US-A- 4 506 010
- APPLIED MICROBIOLOGY, vol. 14, no. 6, November 1966, US; G.S. RAUTELA et al., pp. 892-898/

## Description

The present invention relates to an instrument and method for simply measuring the activity of an enzyme or the activity of an enzymatic reaction-inhibiting substance. The present invention is particularly useful for the measurement of activity of enzymes or the activity of enzymatic activityinhibiting substances in living body samples in the field of clinical inspection.

For the measurement of enzymatic activities, determination of a decomposition substance of a substrate is widely carried out, which has been obtained by reacting the substrate with the enzyme. For example, a method for measuring absorbance of a particular decomposition substance from a substrate, a method for measuring a radioactive isotope in decomposition substances from a substrate labelled with the isotope, a method for the assay of a particular functional group in decomposition substances from a substrate, etc. have heretofore been known as the measurement of enzymatic activities.

In particular, EP-A-0 329 190 discloses an apparatus for the measurement of enzyme activity wherein the enzyme is introduced into a column comprising a hollow tube packed with a filler comprising a support and a substrate that can be recognized by the enzyme, which is immobilized on the support. The measuring is carried out by an detection of the amount of the obtained decomposition product of the substrate.

In "Applied Microbiology, Nov. 1966, pages 892-898", a method for determining the relativ cellulolytic activity of fungi is described, wherein opaque columns of an agar medium containing a partially crystalline cellulose preparation were inoculated with the fungi. Determination is carried out by measurement of the depth (diameter) of the clear zone formed.

The principle of measurement of enzymatic activity in the present invention resides in bringing a sample liquid containing an enzyme into. contact with a substrate gel for use in enzymatic reaction in a thin tube to cause the contemplated enzymatic reaction, measuring the volume of the substrate gel a part of which is dissolved as a result of the enzymatic reaction, and making assay of enzymatic activity based on the result of measurement.

The principle of measurement of the activity of an enzymatic reaction-inhibiting substance in the present invention resides in bringing a sample liquid containing an unknown amount of the enzymatic activity-inhibiting substance to be measured and a given amount of an enzyme into contact with a substrate gel for use in the enzymatic reaction in a thin tube thereby causing the enzymatic reaction, measuring the volume of the substrate gel a part of which is dissolved as a result of the enzymatic reaction, and making assay of the activity based on the result of measurement. In this case, the activity of the enzymatic activity-inhibiting substance is represented by a value obtained by deducting the actually measured enzymatic activity from the enzymatic activity which would be obtained from the given amount of the enzyme.

As the enzymatic reaction is carried out. in a thin tube, the measurement of a substrate gel can easily be made in the present invention.

Any of the substrate gels can be used for the present invention so far as they can react with a particular enzyme selected and can be dissolved in a liquid used. Illustrative of such substrate gel are, for example, conventionally known gelled proteins such as collagen and fibrin. The enzyme as an object of measurement of its activity is selected in relation to the substrate gel. For example, a protease is an object enzyme for gelled proteins. More particularly, collagenase is selected for a substrate gel comprising collagen while plasmin for a substrate gel comprising fibrin.

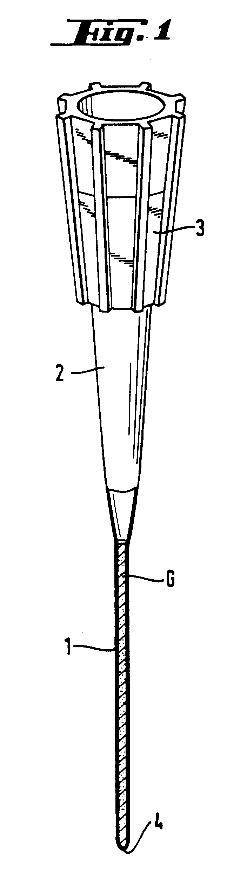

An instrument used for practice of the present invention comprises a thin tube section for being charged with a substrate gel for use in the intended enzymatic reaction and a receptacle section for a sample liquid to be measured which is fitted to one end of the thin tube section. Fig. 1 is a perspective view showing one example of the instrument wherein the reference numeral 1 is a thin tube section charged with a substrate gel G and is provided on the upper end thereof with a receptacle section 2 in the form of a thick tube for a sample liquid to be measured, which is shaped integrally with the thin tube section, 3 is an attachment for adapting itself to a pipetter formed on the upper part of the receptable section 2, and 4 is a sealed end portion at the lower end of the thin tube section. The attachment 3 may be omitted.

In order to charge the substrate gel G into the thin tube section 1, the attachment 3 is mounted to the pipetter through which the liquid substrate is sucked into the thin tube section. The lower end of the thin tube section 1 is then sealed and the liquid substrate is gelled in the thin tube section. A proper method can be used for the gelation of the liquid substrate according to the sort of substrate. Example 1

Basement membrane matrigel (trade name) containing Collagen Type IV as a predominant ingredient (manufactured by Collaborative Research, U.S.A., Catalog. No. 40234) was maintained at normal temperature to convert it into a solution and was taken up in a thin tube section 1 (inner diameter: 0.5 mm, length: 2,5 cm) having the structure as shown in Fig. 1 (manufactured by Quality, U.S.A., Catalog. No. 010). The lower end of the thin tube section 1 was heat-sealed by fusion. The liquefied collagen was solidified (gelled) by allowing it to stand at room temperature for about one hour. The volume of the liquid necessary. to fill the thin tube section was about 4 µ l.

Dispase (trade name) manufactured by Godo Shusei KK which is a protease originated from Bacillus polymyza and having the activity of collagenase type IV was diluted to various concentrations. In the receptacle section 2 was placed 50 µ l of a diluted Disperse solution as a sample liquid to be measured and the substrate G was overlaid with the sample liquid which was then allowed to stand for 72 hours at 37 °C to effect reaction. The sample liquid and the dissolved substrate gel were separated and the length of the remaining substrate gel was measured. A result of the measurement is shown in Table 1. A similar experiment was carried out in the same manner using trypsin, a result of which is shown in Table 2.

**Table 1**

| enzyme (µg/ml) concentration | the length of the remaining gel (mm) |
|---|---|
| 62.5 | 4.60 |
| 31.25 | 3.60 |
| 15.6 | 2.90 |
| 7.8 | 1.50 |
| 3.8 | 1.10 |
| 1.9 | 0.85 |
| 1 | 0.40 |

**Table 2**

| enzyme concentration(µg/ml) | the length of the remaining gel (mm) | |
|---|---|---|
| (µ g/ml) | first | second |
| 2000 | 22.60 | 22.10 |
| 1000 | 21.80 | 20.70 |
| 500 | 18.80 | 20.30 |
| 250 | 15.60 | 16.20 |
| 125 | 14.40 | 14.70 |
| 62.5 | 11.20 | 11.10 |
| 31.25 | 5.50 | 6.20 |
| 15.6 | 0.80 | 1.00 |
| 7.8 | 0.10 | 0.60 |

## Claims

1. An instrument for measuring the activity of an enzyme or the activity of an enzymatic reaction-inhibiting substance, which comprises a thin tube section charged with a substrate gel for an enzymatic reaction and provided on one end thereof with a receptacle section for a sample liquid to be measured by measuring the volume of the substrate gel partially dissolved after the enzymatic reaction.

2. An instrument according to claim 1, wherein the thin tube section has a constant inner diameter.

3. A method for measuring the activity of an enzyme, which comprises charging a thin tube with a substrate gel for an enzymatic reaction, bringing a sample liquid to be measured into contact with the substrate gel in the thin tube thereby causing the enzymatic reaction, and measuring the volume of the substrate gel partially dissolved after the reaction.

4. A method for measuring the activity of an enzymatic reaction-inhibiting substance, which comprises charging a thin tube with a substrate gel for an enzymatic reaction, bringing a sample liquid to be measured which contains an enzymatic reaction-inhibiting substance and a given amount of an enzyme into contact with the substrate gel in the thin tube thereby causing the enzymatic reaction and measuring the volume of the substrate gel partially dissolved after the reaction.

## Patentansprüche

1. Vorrichtung zum Messen der Aktivität eines Enzyms oder der Aktivität einer eine enzyrnatische Reaktion hemmenden Substanz, welche einen dünnen Röhrchenabschnitt aufweist, der mit einem Substratgel für eine enzymatische Reaktion gefüllt und an einem Ende mit einem Behälterabschnitt für eine durch Messen des Volumens des Substratgels, welches nach der enzymatischen Reaktion teilweise aufgelöst ist, zu messende Probenflüssigkeit ausgestattet ist.

2. Vorrichtung nach Anspruch 1, bei welcher der dünne Röhrchenabschnitt einen konstanten Innendurchmesser hat.

3. Verfahren zum Messen der Aktivität eines Enzyms, welches das Befüllen eines dünnen Röhrchens mit einem Substratgel für eine enzymatische Reaktion umfaßt, das Inkontaktbringen einer zu messenden Probenflüssigkeit mit dem Substratgel in dem dünnen Röhrchen, wodurch die enzymatische Reaktion bewirkt wird, und das Messen des Volumens des Substratgels, welches nach der Reaktion teilweise aufgelöst ist.

4. Verfahren zum Messen der Aktivität einer eine enzymatische Reaktion hemmenden Substanz, welches das Befüllen eines dünnen Röhrchens mit einem Substratgel für eine enzymatische Reaktion umfaßt, das Inkontaktbringen einer zu messenden Probenflüssigkeit, welche eine eine enzymatische Reaktion hemmende Substanz und eine vorgegebene Menge eines Enzyms enthält, mit dem Substratgel in dem dünnen Röhrchen, wodurch die enzymatische Reaktion bewirkt wird, und das Messen des Volumens des Substratgels, welches nach der Reaktion teilweise aufgelöst ist.

## Revendications

1. Instrument de mesure de l'activité d'une enzyme ou de l'activité d'une substance inhibant la réaction enzymatique, qui comprend une section de tube mince chargée d'un gel formant substrat pour une réaction enzymatique et est pourvu, sur une de ses extrémités, d'une section formant réceptacle pour un échantillon de liquide à mesurer, par mesure du volume du gel formant substrat partiellement dissous après la réaction enzymatique.

2. Instrument selon la revendication 1, dans lequel la section de tube mince a un diamètre interne constant.

3. Procédé de mesure de l'activité d'une enzyme, comprenant les étapes consistant à charger un tube mince avec un gel formant substrat pour une réaction enzymatique, à amener un échantillon de liquide à mesurer en contact avec le gel formant substrat dans le tube mince, en sorte de provoquer la réaction enzymatique, et à mesurer le volume du gel formant substrat partiellement dissous après la réaction.

4. Procédé de mesure de l'activité d'une substance inhibant la réaction enzymatique, comprenant les étapes consistant à charger un tube mince d'un gel formant substrat provenant d'une réaction enzymatique, à amener un échantillon de liquide à mesurer qui contient une substance inhibant la réaction enzymatique et une quantité donnée d'une enzyme en contact avec le gel formant substrat dans le tube mince, ce qui a pour effet de provoquer la réaction enzymatique, et à mesurer le volume du gel formant substrat partiellement dissous après la réaction.
